# EUROPÄISCHE PATENTSCHRIFT

(11) **EP 0 613 634 B1**
(45) Veröffentlichungstag und Bekanntmachung des Hinweises auf die Patenterteilung: **07.01.1998**
(21) Anmeldenummer: 94102024.0
(22) Anmeldetag: 10.02.1994
(51) Int. Cl.: A45D 40/20, A45D 40/16, B43K 19/16

(54) **Verfahren zur Herstellung von Stiften, insbes. von Kosmetikstiften**
Process for producing sticks, especially cosmetic sticks
Procédé de fabrication de bâtons, en particulier de bâtons cosmétiques

(30) Priorität: 22.02.1993 DE 4305369
(43) Veröffentlichungstag der Anmeldung: 07.09.1994
(73) Patentinhaber: Schwan-STABILO Cosmetics GmbH & Co., 90562 Heroldsberg (DE)
(72) Erfinder: Griebel, Ulrich, D-84032 Altdorf (DE); Schielein, Peter, D-90469 Nürnberg (DE); Kraska, Claudia, D-90409 Nürnberg (DE)
(74) Vertreter: LOUIS, PÖHLAU, LOHRENTZ & SEGETH

(56) Entgegenhaltungen:
- DE-A- 2 834 479
- DE-A- 3 137 486
- DE-C- 2 759 610
- DE-C- 2 759 856
- DE-U- 8 504 263
- GB-A- 1 478 202
- US-A- 1 638 002

## Beschreibung

Die Erfindung betrifft ein Verfahren zur Herstellung von Stiften, insbes. von Kosmetikstiften, mit einer Mine, wobei in einem ersten Verfahrensschritt ein dünnwandiges Röhrchen aus einem spitzbaren Kunststoffmaterial in einem Schaft des Stiftes angeordnet wird, und danach vom einen Ende des Röhrchens her in dieses eine Minenmasse eingebracht wird.

Zur Herstellung von Stiften, insbes. Kosmetikstiften, ist es bekannt, entweder eine geeignete kosmetische Minenmasse kalt zu extrudieren und entsprechend den aus der Bleistift-Produktion bekannten Verfahren in genutete Brettchen einzuleimen, oder gießfähige kosmetische Massen heiß in vorgefertigte Schafthülsen mit konzentrischem Loch einzugießen und die Minenmasse im Schaft erkalten zu lassen.

Bei der zuerst genannten Extrusion sind nur Minenmassen einsetzbar, die zu relativ harten Minen führen, da die Minen zur weiteren Verarbeitung handhabbar bleiben müssen. Bei Minen für Kosmetikstifte ergibt sich hierbei jedoch eine Beschränkung in der Auswahl extrudierbarer Texturen bzw. ein relativ harter Abstrich, der bei kosmetischen Anwendungen nicht erwünscht ist.

Bei dem oben genannten Gießverfahren ergibt sich der Mangel, daß komplett fertiggestellte, d.h. vollständig ausgeformte und dekorierte Schafthülsen angewandt werden müssen, wobei die hierfür erforderlichen Bearbeitungskosten im Falle eines Produktionsausschusses einen die Herstellungskosten beeinflussenden Verlust darstellen. Desweiteren führen bei Verwendung von Hülsen aus Holz-, Holzersatzstoffen oder aus Kunststoff Rauhigkeiten der Innenoberfläche des zentralen Schaftloches beim Eingießen einer heißen Kosmetikmasse durch den sog. Kochlöffeleffekt zur Bildung von Gasblasen. Diese Gasblasen erzeugen in der fertigen Mine Hohlräume, die eine Qualitätsminderung des Stiftes zur Folge haben, die im Extremfall einen Produktionsausschuß darstellt. Desweiteren ist es möglich, daß im verflüssigten Zustand der Minenmasse Bestandteile der Minenmasse vom Material des Schaftes aufgenommen, d.h. absorbiert, werden, wodurch sich eine einen Mangel darstellende Qualitätsänderung der Minenmasse ergeben kann.

Werden kosmetische Minenmassen mit flüchtigen Bestandteilen wie bspw. flüchtigen Silikonen, Paraffinen o.dgl. in Schafthülsen aus Holz oder aus Holzersatzstoffen eingegossen, so ist es erforderlich, die Innenoberfläche des Schaftloches geeignet abzudichten bzw. zu beschichten, um eine Migration bzw. Diffusion der besagten flüchtigen Bestandteile zu verhindern, welche wiederum in unerwünschten Qualitätsänderungen der Mine resultieren würden. Deshalb ist es bislang nicht möglich, flüchtige Bestandteile wie flüchtige Silikonöle, Paraffine o.dgl. enthaltende Kosmetikminen herzustellen und über längere Zeit zwischenzulagern, weil die flüchtigen Bestandteile bei einer Lagerung der Kosmetikminen unkontrollierbar aus den Minenmassen verdunsten, woraus eine unerwünschte Änderung der Minenqualität resultiert.

Ein Verfahren zur Herstellung einer Mine, eine nach diesem Verfahren hergestellte Mine sowie ein Stift mit einer derartigen Mine der eingangs genannten Art ist aus der DE 38 35 680 A1 bekannt. Dort kommt eine Form aus einem Kunststoffmaterial zur Anwendung, welche wenigstens längs eines Teilabschnittes ihrer Mantelfläche schlitzförmig offen ist. Vorzugsweise ist dort die Form mit einem Längsschlitz ausgebildet, der sich über die gesamte Länge der Form erstreckt. Auf diese Weise ist es zwar möglich, durch den Schlitz eine Minenmasse einzubringen, durch die geschlitzte offene Form ist jedoch nicht zu vermeiden, daß flüchtige Bestandteile der Minenmasse insbes. bei einer längeren Zwischenlagerung unkontrollierbar aus der Minenmasse verdunsten, woraus - wie oben erwähnt worden ist - eine unerwünschte Änderung der Minenqualität resultieren kann.

Ein insbes. für Kosmetikzwecke bestimmter Stift mit einer im Gießverfahren hergestellten Mine, die von einem spitzbaren Holzschaft fest umschlossen und gehalten ist, ist aus der DE 27 18 957 C3 der Anmelderin bekannt. Dort ist der Holzschaft als einstückiger Rohrkörper ausgebildet, dessen Innenoberfläche mit einer Beschichtung versehen ist, die als Sperre gegen das Eindringen von Bestandteilen der Minenmasse in den Holzschaft wirkt. Die Minenmasse wird in den rohrförmigen Holzschaft unter Ausbildung einer freiliegenden Minenspitze eingegossen.

Ein Kosmetikstift mit einer im Gießverfahren hergestellten Mine, mit einem durch einen Rohrkörper gebildeten Schaft, der die Mine fest umschließt, wobei der Schaft und die Mine spitzbar sind, mit einer freiliegenden Minenspitze, die eine kegelförmige Mantelfläche aufweist, und mit einem kegelstumpfförmigen Schaftende, das sich an die kegelförmige Mantelfläche der freiliegenden Minenspitze bündig anschließt, ist aus der DE 27 59 610 C2 der Anmelderin bekannt. Bei diesem bekannten Kosmetikstift ist der Schaft ein nahtloser Kunststoffkörper, in welchen die Minenmasse unter Ausbildung der freiliegenden Minenspitze eingegossen ist, wobei sich beim Eingießen der Minenmasse die kegelförmige Mantelfläche sowie ein kalottenförmig abgerundetes Ende der Minenspitze ausbilden. Auch bei diesem bekannten Kosmetikstift kann die Innenoberfläche des Schaftes mit einer Beschichtung versehen sein, durch welche das Eindringen von Minenmasse bzw. entsprechender Bestandteile derselben in den Schaft verhindert wird.

Ein dem zuletzt erwähnten Kosmetikstift ähnlicher Kosmetikstift ist in der DE 27 59 856 C2 der Anmelderin offenbart.

Die DE 40 03 288 A1 beschreibt einen Schreib- oder Malstift mit einer Umhüllung und einer in dieser angeordneten Mine sowie gegebenenfalls weiterer Elemente, wobei die Umhüllung ganz oder überwiegend aus Papiermache, Holzmehl und/oder Holzschliff besteht. Die DE 40 03 289 A1 beschreibt einen Schreib- oder Malstift mit einem Schaft aus einer Umhüllung aus blatt- oder folienförmigen Papier-, Holz- oder Kunststofflagen und einer hierin angeordneten Mine, oder einem sonstigen Schreibmittel und/oder Schreibmittelspeicher, sowie gegebenenfalls Klemm- und/oder Vorschubvorrichtungen und/oder weiteren Elementen, wobei die den Schaft bildende Umhüllung ganz oder überwiegend aus mehrschichtig übereinander angeordneten oder gerollten blatt- oder folienförmigen Papier-, Holz- und/oder Kunststofflagen besteht. Bei diesen Stiften handelt es sich jedoch um Schreib- oder Malstifte und nicht um Kosmetikstifte.

Ein Verfahren der eingangs genannten Art ist aus der GB 15 38 188 bekannt. Das Röhrchen weist dort bspw. einen Innendurchmesser von 2 bis 15 mm und eine Wanddicke von 0,8 bis 3 mm auf, damit das Röhrchen bzw. die Mine, die gleichzeitig ohne weiteres Teil den Kosmetikstift bildet, handhabbar ist. Ein das Röhrchen umgebender Schaft z.B. aus Holz ist bei diesem bekannten Kosmetikstift nicht vorgesehen. Infolge der geringen Wanddicke der Hülse dieses bekannten Kosmetikstiftes ist das Nachspitzen mit einem handelsüblichen Spitzer eine heikle Angelegenheit, wie in dieser Druckschrift ausgeführt wird. Deshalb wird dort nicht nur ein Kosmetikstift sondern auch ein für diesen Kosmetikstift geeigneter spezieller Spitzer vorgeschlagen.

Aus der FR 2 194 113 ist es bekannt, in eine Form eine kosmetische Masse hineinzuextrudieren. Nach der Durchführung des Extrudiervorgangs wird die Form von der gefromten Mine entfernt und die geformte Mine mit einem an sich bekannten Lippenstiftgehäuse kombiniert.

Die DE-AS 2 834 479 offenbart einen kosmetischen Stift mit einer Fettmine, die in einer Holz- oder Kunststoffhülse eingebettet ist. Die Fettmine enthält Parfümstoffe, und zwischen der Fettmine und der Holz- bzw. Kunststoffhülse ist eine metallische Ummantelung vorgesehen. Dort wird explizit ausgeführt, daß die z.B. ätherische Öle enthaltende Fettmine in einer Holz- oder Kunststoffhülle eingebettet ist. Dabei ist die Fettmine mit einer metallischen Ummantelung versehen. Das bedeutet jedoch, daß zuerst die Fettmine vorhanden sein muß, die dann mit der metallischen Umhüllung versehen wird.

Aus der DE 31 37 486 A1 ist ein Stift bzw. ein Verfahren der eingangs genannten Art zur Herstellung eines Stiftes bekannt, bei dem eine Minenmasse in ein Röhrchen aus einem spitzbaren Kuntstoffmaterial vom einen Ende des Röhrchens her in dieses eingebracht wird. Dort ist das besagte Röhrchen mit einer äußeren Schicht bedeckt, die für Lösungsmittel undurchdringlich ist. Die Außenschicht kann ein Kunststoffilm z.B. aus Polyester sein.

Der Erfindung liegt die Aufgabe zugrunde, ein Verfahren der eingangs genannten Art zu schaffen, mit dem es einfach und in kurzen Taktzeiten möglich ist, Stifte, insbes. Kosmetikstifte, herzustellen, wobei die zur Anwendung gelangenden Röhrchen sehr dünnwandig sein können, ohne daß bei ihrer Befüllung mit der Minenmasse Beschädigungen der Röhrchen zu befürchten sind.

Diese Aufgabe wird erfindungsgemäß dadurch gelöst, daß das Röhrchen in einem mit einem Sackloch ausgebildeten Schaft angeordnet wird, wobei das Röhrchen in den Schaft so weit eingebracht wird, daß die dem Grund des Sackloches zugewandte Stirnfläche des Röhrchens vom Grund des Sackloches einen Abstand aufweist, so daß die besagte Stirnfläche des Röhrchens für die Minenmasse eine Halteschulter bildet.

Erfindungsgemäß kann der Schaft aus Holz, einem Holzersatzstoff oder aus einem Kunststoffmaterial bestehen, wobei der mit dem dünnwandigen Kunststoffröhrchen bestückte Schaft einen sog. Kombinationsschaft bildet, in welchen dann die entsprechende Minenmasse eingebracht bzw. vorzugsweise eingegossen wird. Vorzugsweise wird eine heiße Minenmasse eingegossen. Es ist auch möglich, das Röhrchen in eine Schiebe- oder Drehmechanik-Einrichtung einzusetzen und im Bedarfsfall das aus der besagten Einrichtung herausbewegte Röhrchen wunschgemäß zu spitzen.

Das erfindungsgemäße Verfahren weist den Vorteil auf, daß ein Verschließen des vom Eingießende entfernten zweiten Endes des Röhrchens durch Verschweißen oder mittels eines Stopfens entbehrlich ist, und daß sich eine zuverlässige Sicherung der Mine gegen ein Herausfallen aus dem Röhrchen bzw. aus dem Kombinationsschaft bestehend aus Röhrchen und Schaft ergibt. Demselben Zweck bzw. einer Verdrehsicherung ist es dienlich, wenn ein an mindestens einem Abschnitt seiner Längserstreckung mit einem reduzierten und von der Kreisform abweichenden Querschnitt ausgebildetes Röhrchen verwendet wird, das in dem entsprechenden Schaft eingebracht wird, bevor dann die Minenmasse in den sog. Kombinationsschaft eingegossen wird.

Weitere Einzelheiten, Merkmale und Vorteile ergeben sich aus der nachfolgenden Beschreibung verschiedener Verfahren zur Herstellung eines Stiftes, insbes. eines Kosmetikstiftes. Es zeigen:
- Fig. 1: abschnittweise in einem Längsschnitt ein dünnwandiges Röhrchen, das einseitig abgeschlossen ist,
- Fig. 2: eine der Fig. 1 ähnliche Schnittdarstellung eines dünnwandigen Röhrchens, das mit einer Minenmasse befüllt und beidseitig abgeschlossen ist,
- Fig. 3: einen Abschnitt eines genuteten Brettchens für eine Anzahl Minen gemäß Fig. 2,
- Fig. 4: abschnittweise längsgeschnitten einen Schaft mit einem in seinem zentralen Loch angeordneten dünnwandigen Röhrchen gemäß Fig. 1,
- Fig. 5: einen Schnitt durch einen mit einem Sackloch ausgebildeten Schaft, in welchem ein dünnwandiges Röhrchen angeordnet ist, und
- Fig. 6: einen Schnitt entlang der Schnittlinie VI-VI in Fig. 5 durch einen sog. Kombinationsschaft aus Schaft und in seinem zentralen Loch angeordnetem dünnwandigem Röhrchen.

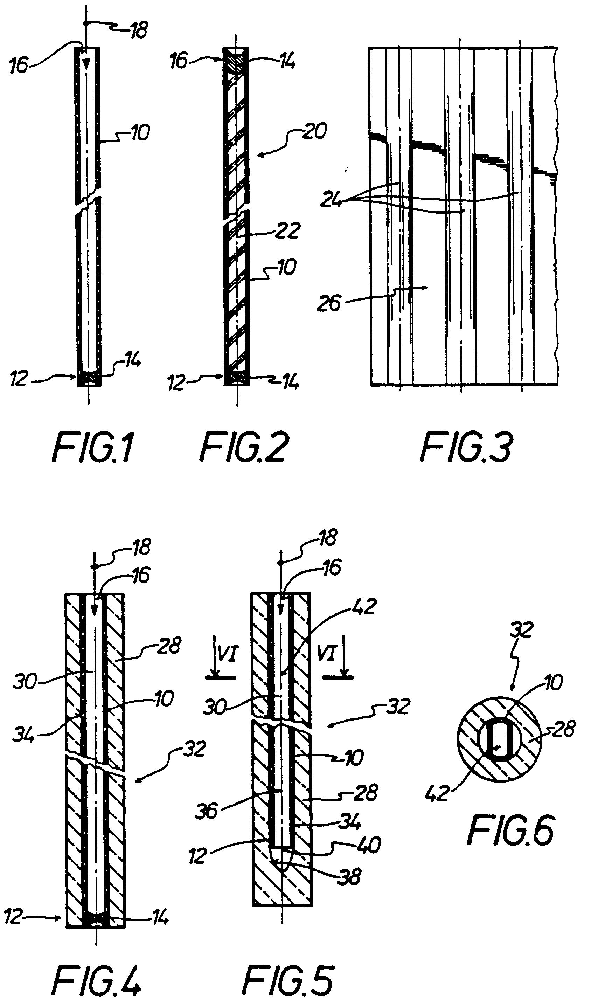

Fig. 1 zeigt ein für eine Mine eines Stiftes, insbes. eines Kosmetikstiftes, vorgesehenes dünnwandiges Röhrchen 10, das aus einem spitzbaren Kunststoffmaterial besteht. Das Röhrchen 10 ist an seinem einen Endabschnitt 12 mittels eines Stopfens 14 abgedichtet. Der Stopfen 14 kann aus einem Kunststoffmaterial, aus Wachs o.dgl. bestehen. Der zweite Endabschnitt 16 des dünnwandigen Röhrchens 10 ist offen, so daß es durch diesen zweiten Endabschnitt 16 hindurch möglich ist, das Röhrchen 10 mit einer Minenmasse, insbes. mit einer kosmetischen Minenmasse, zu befüllen. Das ist in Fig. 1 durch den Pfeil 18 angedeutet. Die kosmetische Minenmasse wird üblicherweise im heißen verflüssigten Zustand in das senkrecht stehende dünnwandige Röhrchen 10 eingegossen.

Fig. 2 zeigt eine Mine 20 mit einer in das dünnwandige Röhrchen 10 eingegossenen Minenmasse 22. Das Röhrchen 10 ist am ersten Endabschnitt 12 und am zweiten Endabschnitt 16 jeweils mittels eines Stopfens 14 abgedichtet, so daß auch nach einer langen Zwischenlagerung der Mine 20 Qualitätsänderungen infolge einer Verdunstung flüchtiger Bestandteile der Minenmasse 22 zuverlässig ausgeschlossen werden. Die auf diese Weise realisierte Mine 20 ist außerdem einfach und problemlos handhabbar, d.h. bspw. in eine Nut 24 eines Brettchens 26 einlegbar bzw. zwischen zwei Brettchen 26 in an sich von der Bleistiftproduktion bekannter Weise einleimbar. Die Fig. 3 zeigt ein solches Brettchen 26 abschnittweise in einer Ansicht von oben. Die einzelnen Nuten 24 können einen halbkreisförmigen Querschnitt oder einen von der Halbkreisform abweichenden Querschnitt besitzen. Bspw. ist es möglich, daß die Nuten 24 einen abgeplatteten oder ovalen Querschnitt aufweisen, um eine gute Klemmung eines Röhrchens (10) bzw. einer Mine (20) zu erzielen.

Fig. 4 zeigt abschnittweise längsgeschnitten einen Schaft 28, der mit einem zentralen Durchgangsloch 30 ausgebildet ist. Im Loch 30 ist ein dünnwandiges Röhrchen 10 aus spitzbarem Kunststoffmaterial festgelegt. Das Röhrchen 10 ist an seinem unteren Endabschnitt 12 mittels eines Stopfens 14 dicht verschlossen. Der zweite Endabschnitt 16 des Röhrchens 10 ist offen, so daß es möglich ist, durch diesen zweiten Endabschnitt 16 hindurch in den Kombinationsschaft 32 aus Schaft 28 und dünnwandigem Röhrchen 10, d.h. in das dünnwandige Röhrchen 10, erfindungsgemäß eine Minenmasse, insbes. eine kosmetische Minenmasse, einzugießen, was in Fig. 4 durch den Pfeil 18 angedeutet ist. Für das dünnwandige Röhrchen 18 wird hierbei vorzugsweise ein Kunststoffmaterial gewählt, das beim Eingießen der heißen verflüssigten Minenmasse erweicht und sich eng an die Innenoberfläche des Loches 30 des Schaftes 28 anlegt, wodurch das mit der Minenmasse befüllte Röhrchen 10 gegen ein Herausfallen aus dem Schaft 28 gesichert ist.

Fig. 5 zeigt einen Kombinationsschaft 32 aus einem Schaft 28 und einem dünnwandigen Röhrchen 10, wobei der Schaft 28 mit einem zentralen Sackloch 36 ausgebildet ist. Das Sackloch 36 ist mit einem sich verjüngenden Grund 38, bspw. mit einem parabolischen Grund 38 oder mit einem sich kegelstumpfförmig verjüngenden Grund 38, ausgebildet. Hierdurch wird die Einsetzbewegung des Röhrchens 10 in das Sackloch 36 hinein definiert derartig begrenzt, daß die dem Grund 38 des Sackloches 36 zugewandte ringförmige Stirnfläche 40 des dünnwandigen Röhrchens 10 vom Grund 38 des Sacklochs 36 einen bestimmten Abstand aufweist. Wird in einen solchen Kombinationsschaft 32 vom offenen Endabschnitt 16 her erfindungsgemäß eine heiße verflüssigte Minenmasse eingegossen, was durch den Pfeil 18 angedeutet ist, so bildet die besagte ringförmige Stirnfläche 40 des dünnwandigen Röhrchens 10 für die Minenmasse eine Halteschulter, durch welche die Minenmasse in bezug auf das Röhrchen 10 fixiert ist. Demselben Zweck ist es dienlich, wenn ein Röhrchen 10 verwendet wird, das an mindestens einem Abschnitt 42 seiner Längserstreckung mit einem reduzierten und von der Kreisform abweichenden Querschnitt ausgebildet ist, wie aus Fig. 6 ersichtlich ist, die einen Schnitt durch den Kombinationsschaft 32 aus Schaft 28 und dünnwandigem Röhrchen 10 entlang der Schnittlinie VI-VI in Fig. 5 zeigt.

Die Länge des Röhrchens 10 kann wunschgemäß dimensioniert, d.h. geeignet an die Länge des Sackloches 36 angepaßt sein. Die Stirnfläche 40 des Röhrchens 10 muß nicht bis zum sich verjüngenden Grund 38 des Sackloches 36 reichen; sie kann hiervon auch beabstandet sein, ohne hierdurch die Fixierung der Minenmasse zu beeinträchtigen.

## Patentansprüche

1. Verfahren zur Herstellung von Stiften, insbes. von Kosmetikstiften, mit einer Mine, wobei in einem ersten Verfahrensschritt ein dünnwandiges Röhrchen (10) aus einem spitzbaren Kunststoffmaterial in einem Schaft (28) des Stiftes angeordnet wird, und danach vom einen Ende (16) des Röhrchens (10) her in dieses eine Minenmasse (22) eingebracht wird,
**dadurch gekennzeichnet,**
daß das Röhrchen (10) in einem mit einem Sackloch (36) ausgebildeten Schaft (28) angeordnet wird, wobei das Röhrchen (10) in den Schaft (28) so weit eingebracht wird, daß die dem Grund (38) des Sackloches (36) zugewandte Stirnfläche (40) des Röhrchens (10) vom Grund (38) des Sackloches (36) einen Abstand aufweist, so daß die besagte Stirnfläche (40) des Röhrchens (10) für die Minenmasse (22) eine Halteschulter bildet.

2. Verfahren nach Anspruch 1,
**dadurch gekennzeichnet**,
daß ein an mindestens einem Abschnitt (42) seiner Längserstreckung mit einem reduzierten und von der Kreisform abweichenden Querschnitt ausgebildetes Röhrchen (10) verwendet wird.

## Claims

1. Method of producing sticks, in particular cosmetic sticks, having a refill, a thin-walled tube (10) made of an injection-mouldable plastic material being arranged in a shank (28) of the stick in a first method step, and then a refill substance (22) being introduced into the tube (10) from one end (16) of the same, characterized in that the tube (10) is arranged in a shank (28) which is formed with a blind hole (36), the tube (10) being introduced into the shank (28) to the extent where the end surface (40) of the tube (10) which is directed towards the base (38) of the blind hole (36) is at a distance from the base (38) of the blind hole (36), with the result that said end surface (40) of the tube (10) forms a retaining shoulder for the refill substance (22).

2. Method according to Claim 1, characterized in that use is made of a tube (10) which, on at least one section (42) of its longitudinal extent, is designed with a reduced cross-section which is not circular.

## Revendications

1. Procédé de fabrication de crayons, en particulier de crayons cosmétiques, dotés d'une mine, dont une première étape consiste à disposer une tubulure (10) à parois minces en matériau synthétique taillable dans un bâton (28) du crayon, et à insérer ensuite à partir de l'une des extrémités (16) de ladite tubulure (10) et dans celle-ci une composition de mine (22), caractérisé en ce que ladite tubulure (10) est disposée dans un bâton (28) doté d'un orifice sans issue (36), dans lequel elle est insérée de sorte que subsiste une distance résiduelle entre le fond (38) de l'orifice sans issue (36) et la surface (40) de ladite tubulure (10) en regard du fond (38), afin que ladite surface (40) constitue un épaulement d'appui pour la composition de mine (22).

2. Procédé selon la revendication 1, caractérisé en ce que la tubulure (10) présente sur au moins une partie (42) de sa longueur une section réduite différente d'une section circulaire.
